# DEMANDE DE BREVET EUROPEEN

(11) **EP 0 856 301 A1**
(43) Date de publication de la demande: **05.08.1998**
(21) Numéro de dépôt: 97116089.0
(22) Date de dépôt: 17.09.1997
(51) Int. Cl.: A61F 6/04

(54) **Prophylactique**

(30) Priorité: 31.01.1997 IT MI970201
(71) Demandeur: Cattaneo, Guido, 21014 Laveno (VA) (IT)
(72) Inventeur: Cattaneo, Guido, 21014 Laveno (VA) (IT)

(57) **Abrégé**

Un prophylactique (10) obtenu en làtice de gomme ou autre materiel approprié, est fourni, tout au long sa superficie extérieure, des moyens (16) etendus longitudinelement, n'emporte l'hauteur et l'épaisseur de la section, laquelle partie anterieure (20) est disposée à la proximité de la partie anterieure (18) du prophylactique même d'où sort au moment où le prophylactique est enveloppé sur soi même.

## Description

Cette invention concerne un prophylactique. En détail, cette invention concerne un prophylactique fourni des accessoires qui permettent à l'utilisateur duprophylactique de l'enfiler facilement et rapidement.

Il est de notoriété publique que ces produits sont traditionnellement employés dans un but d'anticonceptionnell et prophylaxie. Surtout pour ce qui concerne la prophylaxie, dans le marché de ces produits on a récemment constaté une bonne augmentation de vente, surtout dû au virus du SIDA. En effet, le prophylactique est l'un des plus importants moyens de prévention. Une importante contribution à ce propos vient des massives campagnes d'informations conduites par les indrustries du secteur et par les moyens de communications, qui ont beaucoup parlé des implications sanitaires de ce produit et qui rencontre des difficultés pour ce qui concerne l'innovation.

D'un côté les condoms qui on connaissent ont eu, avec les temps, des perfectionnements importants qui proviennent surtout de procédés productifs qui ont perfectionné les marges de sécurité. De l'autre côté, en comparaison de ces avantages, on a encore des grands problèmes pour ce qui concerne la manipulation et l'emploi des condoms, problèmes que donnent de la gêne et peuvent se répercuter au niveau psychologique.

Il s'agit, par exemple, de la difficulté d'application du prophylactique qui doit être manipulé pendant le déroulement pour pouvoir être rapidement enfilé; conditions de lumière faible ou problèmes de vue de l'utilisateur peuvent facilement compliquer l'action et, en certain cas, l'obliger de remplacer le prophylactique.

Autre inconvénient est lié à la nature glissante de ces condoms, souvent recouvert de substances lubrifiantes qui entravent la facile manipulation pendant l'application et, quelque fois, provoquent une inversion dans le sens du déroulement des condoms.

But de cette invention est esseyer de obvier aux inconvénients avant décrit.

En particulier, le but de cette invention est la réalization d'un prophylactique que peut être manipulé et erfilé trés rapidement, dans toutes conditions.

Autre but de l'invention est mettre à la disposition un prophylactique, comme ci-dessus mentionné, qui peut garantir le traditionell niveau de sécurité et être aussi facilement et économiquement réalisé.

Le prophylactique de cette invention atteint cet but et d'autre aussi, parce que est obtenu en làtice de gomme ou autre materiel approprié. Il est fourni, dans le sens de la longueur à l'extérieur, des moyens longitudinalement étendus, n'importe quelle hauteur ou épaisseur, dont la partie anterieure se trouve à proximité de la partie à l'avant du prophylactique même de laquelle sort au moment où le prophylactique est enveloppé sur soi même.

Les carachtéristiques de la construction et les fonctions du prophylactique de cette invention peuvent être mieux comprises avec la description suivant où on fait référence à la table de la figure que en répresentent la forme d'execution préférée et pas limitative et où:
**figure 1** répresent schématiquement, latéralement, le prophylactique de cette invention comme se trouve avant l'enveloppment sur soi même
**figure 2** répresent le prophylactique tout à fait enveloppé sur soi même et préparé à confectionner
**figure 3** réoresent schématiquement le même prophylactique, en partie, enfilé sur l'organe génital
**figure 4** répresent schématiquement un détail du prophylactique enveloppé sur soi même, pour montrer la position des moyens qui permettent de dérouler rapidement le prophylactique
**figure 5** répresent schématiquement le prophylactique tout à fait enfilé sur l'organe génital
**figure 6** répresent schématiquement une solution alternative du même prophylactique, où les moyens de déroulement sont unis entre eux

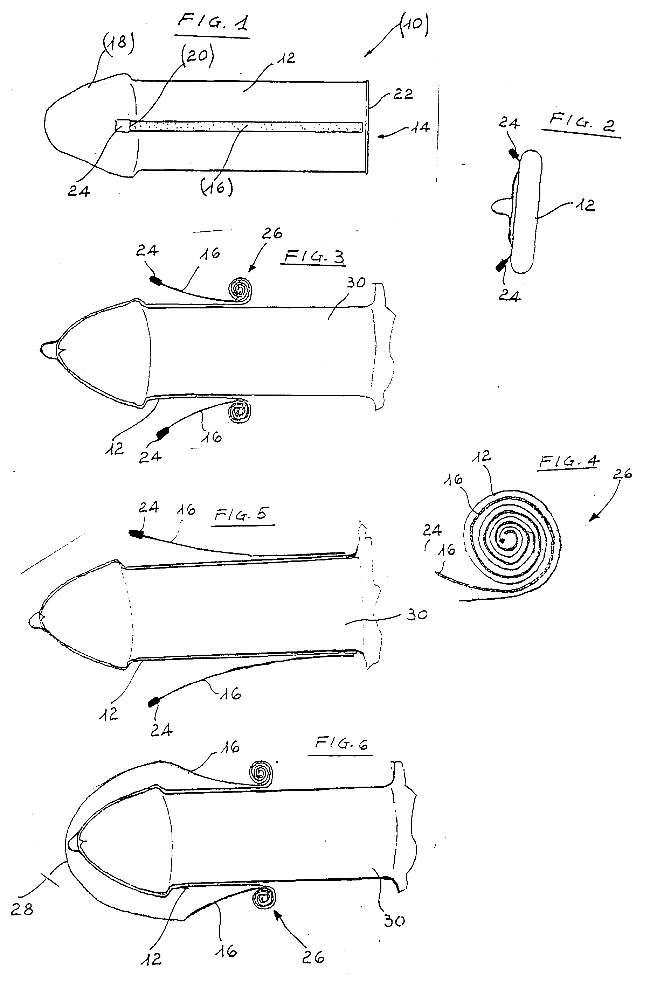

En référence aux figures, le prophylactique de cette invention, indiqué dans la figure 1 avec le n. 10, obtenu in làtice de gomme ou autre materiel approprié, est par convention constitué par un enveloppe cylindrique (12) avec front (14) ouvert, selon la répresentation schématique de la figure 1 que lui montre dans sa forme complete et étendue avant de l'enrouler.

Selon cette invention, le prophylactique (10) est avantageusement fourni des moyens qui permettent de le dérouler rapidement pendant l'application, pour eviter à l'utilisateur les inconvenients ci-dessus mentionnés.

Ces moyen sont préférablement constitués d'une ou plus bandes (16) de bas épaisseur, n'emporte le materiel (par exemple papier imperméable, plastique ou équivalent) appliquées ou approchées au front éxtérieur (10) à proximité de sa partie supérieure à l'avant, indiquée avec 18. Selon une forme d'exécution pas encore determinée, à cette partie 18 on applique ou on approche le bord 20 que définit la partie à l'avant d'une couple des bandes opposées 16 étendues longitudinalement et parallélement sur le condom 10 jusqu'à la partie postérieure ouverte à la fin du condom 14 ou bien à l'approche de la même partie, qui termine avec un collier traditionel et solide 22. Les extrémités de ces bandes 16 sont avantageusement et pas obligatoirement fournies de moyens de prise 24, qui ont un groissement des bandes mêmes ou d'autres elements liés aux bandes mêmes, n'emporte la forme et la dimension, obtenues en plastique ou autre materiel approprié.

Figure 3, et plus en détail figure 4, montrent la position que les bandes mêmes 16 prennent au moment que le condom s'enveloppe sur soi même 10, avant de le confectionner. Ce prophylactique enveloppé sur soi même forme un noyau en spiral 26, où le materiel du condom 10 est espacé d'une ou plus bandes 16; terminé l'enveloppment, débordent de cet noyan 26 les éxtrémites 20 des cidessus mentionnées bandes fournies des moyens de prise 24. Les bandes 16 peuvent presenter n'emporte quelle hauter et épaisseur, mais on préfére solutions où ces mesures sont les plus possible réduites; le front à l'avant 20 des mêmes bandes peut être lié à la partie à l'avant 18 du prophylactique 10 par des substances compatibles avec le materiel du prophylactique même, qui ne changent pas les caracteristiques d'herméticité.

On préfére la solution selon la quelle ces bandes sont seulement approchées et/ou comprimées sur l'enveloppe 12 avant l'enroulement; selon cette hypothése, l'adhésion pas définitive des bandes 16 est facilitée par des substances lubrifiantes, qui on connaît déjà et conventionellment employées, étalées sur le meme enveloppe 12.

Figure 6 montre schématiquement une autre forme d'exécution alternative du prophylactique de cette invention, selon laquelle les bandes 16 sont liées entre elles à la partie à l'avant 20 par un semi-anneau 28, lié aux bandes même, qui serve comme prise unique pour le déroulement du prophylactique 10; selon cette hypothése les prises ne sont pas necessaires à l'extrémités des bandes 16. Le semi- anneau 28, dans la répresentation grafique dont à la figure 6, est orienté, pour une meilleure compréhension sur le front anterieur du prophylactique 10, mais il est évident que, pendant l'application, le prophylactique vient poussé et conduit vers la direction opposée.

L'emploi du prophylactique de cette invention on peut facilement déduire de ce ci-dessus decrit, en référence aux figures annexées; l'enveloppe 12 s'enfile facilement
sur l'organe génital, figure 30 pour la possibilité de son déroulement rapide à partir des prises 24 des bandes 16, ou bien à partir du semi-anneau 28 que unis les extrémités à l'avant 20 des mêmes bandes; à la fin de l'application, les bandes sont automatiquement éliminées, parce que préférablement pas liées d'une maniére durable à l'enveloppe même.

Cette invention, comme ci-dessus decrit à été proposée comme exemple, et cela s'entend que la même est susceptible d'être développé avec modification et variantes.

Par exemple, le numero des bandes 16, leur éxtension, orientation et mesures de la section pourrons résulter differents par rapport à ce que on a ci-dessus decrit et illustré; les mêmes bandes libres ou liées au prophylactique 10 pourront être eventuellement liées dans le sens transversale entre elles, dans un ou plus points, et être obtenues en forme de filaments, (n'emporte la section), de materiel différent par exemple patês de silicone, patês caoutchouteuses ou similaires.

On doit aussi prévoir que ces bandes 16 peuvent être remplacées par un manchon en làtice de gomme, plastique, papier ou autre materiel approprié, qui s'enleve ou s'elimine avec un coup sec aprés l'application du prophylactique 10.

Ce sont possibles, encore, des inversions de structure éventuelles ou déplacements alternatifs des parties que dans l'ensemble forment le prophylactique de cette invention.

## Revendications

1. Prophylactique (10) obtenu en làtice de gomme ou autre materiel approprié fourni tout au long sa superficie éxterieure des moyens (16) etendus longitudinalement, n'emporte l'hauteur épaisseur et section, dont la partie à l'avant (20) se trouve prés de la portion à l'avant (18) du prophylactique même, d'où sort au moment où le prophylàctique est enveloppé sur soi même

2. Prophylactique selon la revendication 1, où ces moyens (16) sont constitués d'une ou plus bandes, obtenues en papier, plastique ou autre materiel approprié, en spirale et alternés à l'enveloppe (12) qui forment le prophylactique même quand il est totalement enveloppé

3. Prophylactique selon les revendications ci-dessus mentionnées, où à cette partie à l'avant (20) des bandes (16) est lié un moyen de prise (24) qui est constitué d'un grossissement de la même partie (20) ou d'un autre pièce de rapport, n'emporte la forme et materiel

4. Prophylactique selon les revendications ci-dessus mentionnées, où les bandes (16) sont deux entre elles opposées, approchées à l'enveloppe (12) avant l'enveloppment du prophylactique sur soi même

5. Prophylactique selon les revendications d'avant, où les extrémités à l'avant (20) de ces bandes sont liées entre elles à former un semi-anneau (28)

6. Prophylactique selon les revendications d'avant, où ces bandes (16) sont liées dans un ou plus points à l'enveloppe 12

7. Prophylactique selon les revendications d'avant, où ces bandes (16) sont liées dans le sens transversale entre elles dans un ou plus points.

8. Prophylactique selon les revendications d'avant, où ces moyens (16) sont constitués d' un manchon, en làtice de gomme, plastique, papier ou autre materiel approprié qui s'enleve ou s'élimine avec un coup sec après l'application du prophylactique même

9. Prophylactique selon les revendications d'avant, où les bandes (16) sont constituées par des filaments en pâte de silicone, gomme ou autre materiel approprié.
